(19) 

Europäisches
Patentamt
European
Patent Office
Office européen
des brevets

(11)  **EP 4 299 716 B1**

(12)  **EUROPEAN PATENT SPECIFICATION**

(45) Date of publication and mention
of the grant of the patent:
**29.04.2026  Bulletin 2026/18**

(21) Application number: **22775693.9**

(22) Date of filing: **23.03.2022**

(51) International Patent Classification (IPC):
*C12M 3/04* (2006.01)      *C08L 25/18* (2006.01)
*C08L 53/00* (2006.01)      *C12M 3/00* (2006.01)
*C12M 1/26* (2006.01)

(52) Cooperative Patent Classification (CPC):
(C-Sets available)
**C12M 23/20; C08L 53/00; C12M 33/00;
C12M 41/12**                                (Cont.)

(86) International application number:
**PCT/JP2022/013590**

(87) International publication number:
**WO 2022/202911 (29.09.2022 Gazette 2022/39)**

(54) **TEMPERATURE-RESPONSIVE POLYMER SURFACE TREATMENT AGENT**

TEMPERATURREAKTIVES POLYMEROBERFLÄCHENBEHANDLUNGSMITTEL

AGENT DE TRAITEMENT DE SURFACE POLYMÈRE THERMOSENSIBLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.03.2021  JP 2021054194
28.04.2021  JP 2021076035**

(43) Date of publication of application:
**03.01.2024  Bulletin 2024/01**

(73) Proprietor: **Tosoh Corporation
Yamaguchi 746-8501 (JP)**

(72) Inventors:
• **JIDO Yumiko
Ayase-shi, Kanagawa 252-1123 (JP)**

• **HIRATOKO Shoya
Ayase-shi, Kanagawa 252-1123 (JP)**
• **IMATOMI Shinya
Ayase-shi, Kanagawa 252-1123 (JP)**
• **ITO Hiroyuki
Ayase-shi, Kanagawa 252-1123 (JP)**

(74) Representative: **Novagraaf International SA
Chemin de l'Echo 3
1213 Onex, Geneva (CH)**

(56) References cited:
**WO-A1-2006/019043      JP-A- 2013 055 907
JP-A- 2018 087 316      JP-A- 2020 110 140
JP-A- 2020 174 660**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08L 53/00, C08L 25/18**

## Description

## Technical Field

The present invention relates to a temperature-responsive polymer surface treatment agent.

## Background Art

Since biogenetic pharmaceuticals containing cells as a raw material have a high medicinal effect, the spread thereof has been expected, and a technology of efficiently culturing the raw material cells has attracted attention. Many useful cells adhere to any substrate and proliferate when cultured, but after proliferation, it is necessary to peel off the cultured cells and perform a subculture operation. In general, an enzyme treatment using a protein-degrading enzyme such as trypsin is performed, but the enzyme treatment has an insufficient efficiency because a complicated operation of removing the enzyme is accompanied.

In order to solve the above problems, in Patent Literature 1, a cell recovery method by a cooling treatment is described. In addition, in Patent Literatures 2 and 3, a cell culture substrate modified with a temperature-responsive polymer is described. By cooling cells cultured using the cell culture substrate covered with the temperature-responsive polymer to a sol transition temperature or lower of the temperature-responsive polymer, the adhesive force of the surface of the substrate is weakened, and it is possible to peel off/recover the cells without requiring a complicated operation. On the other hand, the temperature-responsive polymer itself has weak cell adhesiveness, and sufficient cell adhesiveness may not be obtained in accordance with a covering amount or the type of cell culture substrate.

Accordingly, a temperature-responsive cell culture substrate making the cell recovery by the cooling treatment and the cell adhesiveness compatible is required.

## Citation List

## Patent Literature

Patent Literature 1: Japanese Unexamined Patent Publication No. 2020-110140
Patent Literature 2: Japanese Patent No. 5846584
Patent Literature 3: Japanese Patent No. 6447787.

The document JP201355907 discloses a surface treatment agent suitable as a coating for cell culture substrates, comprising a temperature-responsive polymer and a cell-adhesive polymer comprising methoxyethyl acrylate and a monomer comprising a carboxyl group.

## Summary of Invention

## Technical Problem

An object of the present invention is to provide a temperature-responsive polymer surface treatment agent.

## Solution to Problem

As a result of intensive studies of the present inventors in consideration of the problems described above, it has been found that by covering a substrate with a surface treatment agent containing a temperature-responsive polymer, and a cell-adhesive polymer including a monomer having a functional group exhibiting acidity and a monomer having a HLB value (a Griffin's method) in a range of 0 to 5.0, a temperature-responsive cell culture substrate making cell adhesiveness and cell recovery by a cooling treatment compatible is obtained, and the present invention has been completed. That is, the present invention includes the following aspects.

<1> A surface treatment agent, containing: a temperature-responsive polymer; and a cell-adhesive polymer including (A) and (B) described below;

(A) a monomer having a functional group exhibiting acidity, and
(B) a monomer having a HLB value (a Griffin's method) in a range of 0 to 5.0.

<2> The surface treatment agent according to <1>, in which an introduced amount of the cell-adhesive polymer is 1 to 50 wt% with respect to an introduced amount of the temperature-responsive polymer.

<3> The surface treatment agent according to <1> or <2>, in which the temperature-responsive polymer exhibits a lower critical solution temperature with respect to water in a range of 0°C to 50°C.

<4> The surface treatment agent according to any one of <1> to <3>, in which the temperature-responsive polymer is a block copolymer including repeating units of three or more components.

<5> The surface treatment agent according to any one of <1> to <4>, in which the functional group exhibiting acidity of the constituent (A) is selected from a hydroxy group, a carboxy group, a sulfo group, and a phosphate group.

<6> The surface treatment agent according to any one of <1> to <5>, in which an acidity dissociation constant pKa of the functional group exhibiting acidity of the constituent (A) is -5.0 to 6.0.

<7> The surface treatment agent according to any one of <1> to <6>, in which a concentration of the temperature-responsive polymer in the surface treatment agent is 0.1 to 5.0 wt%.

<8> A film obtained by applying the surface treatment agent according to any one of <1> to <7> to a substrate.

<9> The film according to <8>, in which a film thickness is 1 to 1000 nm.

<10> A cell culture substrate having a surface covered with the film according to <8> or <9>.

<11> The cell culture substrate according to <10>, in which the cell culture substrate is in the shape of a film.

<12> A method for manufacturing the cell culture substrate according to <10> or <11>, the method including a step of applying the surface treatment agent to the substrate.

**Advantageous Effects of Invention**

By covering the substrate with the surface treatment agent containing the temperature-responsive polymer, and the cell-adhesive polymer including the monomer having a functional group exhibiting acidity and the monomer having a HLB value (a Griffin's method) in a range of 0 to 5.0, the temperature-responsive cell culture substrate making the cell adhesiveness and the cell recovery by the cooling treatment compatible is obtained.

**Description of Embodiments**

Hereinafter, embodiments for carrying out the present invention (hereinafter, simply referred to as "this embodiment") will be described in detail. The embodiments described below are an example for describing the present invention, and are not intended to limit the present invention to the following contents. The present invention can be suitably modified and carried out within the scope of the gist thereof. In the present invention, a "surface treatment agent" may be referred to as a "polymer solution".

The surface treatment agent of the present invention is a surface treatment agent containing a temperature-responsive polymer, and a cell-adhesive polymer including a monomer having a functional group exhibiting acidity and a monomer having a HLB value (a Griffin's method) in a range of 0 to 5.0.

The temperature-responsive polymer of this specification is not particularly limited, and preferably exhibits a lower critical solution temperature in a range of 0°C to 50°C. More preferably, the temperature-responsive polymer exhibits the lower critical solution temperature in a range of 22°C to 37°C. Examples of a repeating unit of a temperature-responsive component having a lower critical solution temperature (LCST) with respect to water and LCST with respect to water may include N-cyclopropyl acrylamide (LCST = 46°C), N-isopropyl acrylamide (LCST = 32°C), N-n-propyl methacrylamide (LCST = 22°C), N-tetrahydrofurfuryl acrylamide (LCST = 28°C), N-ethoxy ethyl acrylamide (LCST = 35°C), N,N-diethyl acrylamide (LCST = 32°C), N-isopropyl methacrylamide (LCST = 44°C), N-n-propyl methacrylamide (LCST = 28°C), N-tetrahydrofurfuryl methacrylamide (LCST = 35°C), N-methyl-N-isopropyl acrylamide (LCST = 23°C), N-methyl-N-n-propyl acrylamide (LCST = 20°C), N,N-dimethyl aminoethyl methacrylate (LCST = 47°C), or the like.

Among them, the repeating unit exhibiting LCST between 37°C, which is the usual cell culture temperature, and 22°C, which is the general room temperature, is preferable, and the N-isopropyl acrylamide is particularly preferable. In addition, different repeating units may be included in addition to the temperature-responsive repeating unit, insofar as the repeating units have LCST. As an example, in a case where the temperature-responsive repeating unit is N-isopropyl acrylamide, LCST when including different repeating units may be exhibited in 0°C to 100°C.

The structure of the temperature-responsive polymer of this specification is not particularly limited, and in the case of including two or more constituents, a block copolymer is preferable in order to exhibit high temperature responsivity. Insofar as the temperature-responsive polymer contains one or more temperature-responsive components, the temperature-responsive polymer may contain components having different properties, and examples thereof include a block copolymer containing styrene and a derivative thereof, 2-methoxy ethyl acrylate, n-propyl acrylate, n-propyl methacrylate, n-butyl acrylate, n-butyl methacrylate, and the like. From the viewpoint of obtaining high temperature responsivity and an effect of improving covering properties with respect to a substrate, a block copolymer containing three components of a block including a repeating unit of 2-methoxy ethyl acrylate, a repeating unit of n-butyl acrylate, and a repeating unit of N-isopropyl acrylamide is particularly preferable.

A composition ratio of the repeating unit of the temperature-responsive component in the temperature-responsive polymer of this specification is not particularly limited, and as an example, is 1 to 100 mol%. In addition, a number average molecular weight Mn of the temperature-responsive polymer is not also particularly limited, and as an example, is 1000 to 1000000.

The functional group exhibiting acidity of the constituent (A) of this specification is a functional group that is ionized in water to exhibit anionicity, is not particularly limited, and as an example, is selected from a hydroxy group, a carboxy group, a sulfo group, and a phosphate group. Two or more of the functional groups may be contained in the monomer, and a functional group other than the functional groups described above, for example, an aldehyde group, a carbonyl group, a nitro group, an amino group, an ether group, an ester group, and an amide group may be contained.

An acidity dissociation constant of this specification indicates an acidity dissociation constant in water. pKa is specified from a side-chain structure of the polymer structure. In order to impart cell adhesiveness, it is necessary to accelerate cell-substrate electrostatic bonding, and the acidity dissociation constant pKa of the functional group exhibiting acidity of the constituent (A) of the present invention is not particularly limited, and is -5.0 to 6.0, and preferably -3.0 to 5.0. In a case where the acidity dissociation constant pKa is less than -5.0, copolymerization with the constituent (B) becomes difficult, and in a case where the acidity dissociation constant pKa is greater than 6.0, an accelerative effect of the electrostatic bonding is degraded. As an example of the acidity dissociation constant, since a side-chain structure of an acrylic acid is -COOH, an acidity constant is 4.76, since a side-chain structure of p-carboxy styrene is $-C_6H_4COOH$, an acidity constant is 4.20, and since a side-chain structure of a p-styrene sulfonic acid is $-C_6H_4SO_3H$, an acidity constant is -2.80.

The constituent (A) of this specification is not particularly limited, and examples thereof include an acrylic acid, vinyl phenol, carboxy styrene, styrene sulfonate, and a derivative thereof.

A HLB value (HLB; hydrophile-lipophile balance) of this specification is a value representing the degree of affinity to water and oil, which is described in W. C. Griffin, Journal of the Society of Cosmetic Chemists, 1, 311 (1949), and is a value of 0 to 20, in which hydrophobicity increases as the value becomes closer to 0, and hydrophilicity increases as the value becomes closer to 20. Examples of a method for determining the HLB value by calculation include an Atlas' method, a Griffin's method, a Davies' method, and a Kawakami's method, and in the present invention, a value calculated by the Griffin's method is used, and the value is obtained by the following calculation expression, on the basis of a formula weight of a hydrophilic part in a repeating unit, and the total formula weight of the repeating unit.

HLB Value = 20 × (Formula Weight of Hydrophilic Part) ÷ (Total Formula Weight)

As the definition of the hydrophilic part in the repeating unit of each block, described above, a sulfo group part ($-SO_3-$), a phospho group part ($-PO_3-$), a carboxy group part (-COOH), an ester part (-COO-), an amide part (-CONH-), an imide part (-CON-), an aldehyde group part (-CHO), a carbonyl group part (-CO-), a hydroxy group part (-OH), an amino group part ($-NH_2$), an acetyl group part ($-COCH_3$), an ethylene amine part ($-CH_2CH_2N-$), an ethylene oxy part ($-CH_2CH_2O-$), an alkaline metal ion, an alkaline earth metal ion, an ammonium ion, a halide ion, and an acetic acid ion can be exemplified.

In the calculation of the hydrophilic part in the repeating unit, it is necessary that atoms configuring the hydrophilic part do not overlap with atoms configuring another hydrophilic part. A calculation example of the HLB value in the repeating unit is described below. For example, in the case of styrene (Molecular Weight: 104.15), since there is no hydrophilic part, and the molecular weight of the hydrophilic part is 0, a HLB value is 0.0. In the case of p-carboxy styrene (Molecular Weight: 148.15), since a hydrophilic part is 1 part of a carboxy group, and the molecular weight of the hydrophilic part is 45.02, a HLB value is 6.1.

In order to impart the cell adhesiveness, it is necessary to accelerate cell-substrate hydrophobic bonding, and the constituent (B) of this specification is a monomer having a HLB value in a range of 0 to 5.0. In a case where the HLB value is greater than 5.0, an accelerative effect of the hydrophobic bonding is degraded.

The constituent (B) of this specification is not particularly limited, and examples thereof include a styrene derivative including styrene, vinyl naphthalene, and vinyl anthracene, olefin, acrylate, and methacrylate.

A ratio of the constituent (A) in the cell-adhesive polymer of this specification is not particularly limited, and is 1 to 99 mol%, and preferably 10 to 50 mol%. In a case where the ratio is less than 10 mol%, solubility to an applicable solvent decreases, and in a case where the ratio is greater than 50 mol%, a balance between the cell-substrate electrostatic bonding and the cell-substrate hydrophobic bonding is lost, and it is difficult to exhibit sufficient cell adhesiveness.

In the cell-adhesive polymer of this specification, a constituent other than the constituent (A) and the constituent (B) may be included, and as an example, alkyl (meth)acrylate may be copolymerized in order to obtain the solubility to the solvent. In addition, the cell-adhesive polymer may be a copolymer in which the constituents are randomly arranged, or may be a block copolymer in which polymers respectively including the constituents are connected.

A number average molecular weight Mn of the cell-adhesive polymer of this specification is not particularly limited, and as an example, is 5000 to 1000000.

By dissolving the temperature-responsive polymer and the cell-adhesive polymer of this specification in a solvent, the surface treatment agent, which is the present invention, is obtained. The solvent of the surface treatment agent is not particularly limited, and it is preferable to select a solvent in which the temperature-responsive polymer and the cell-adhesive polymer are soluble, and the substrate to be covered is insoluble, and as an example, methanol, ethanol, 1-propanol, 2-propanol, 1-butanol, 2-butanol, t-butanol, acetone, methyl ethyl ketone, diethyl ether, tetrahydrofuran, dimethyl formamide, n-hexane, benzene, toluene, 1,4-dioxane, 2-methoxy ethanol, 1-methoxy-2-propanol, 3-methoxy-1-butanol, and a mixed liquid selected therefrom can be used.

The concentration of the temperature-responsive polymer in the surface treatment agent of the present invention is not particularly limited, and an arbitrary concentration can be selected in accordance with a film formation method or a target film thickness, and as an example, the concentration is 0.1 to 5.0 wt%, and preferably 0.2 to 3.0 wt%. In addition, a compound other than the temperature-responsive polymer and the cell-adhesive polymer of the present invention may be contained.

An introduced amount of the cell-adhesive polymer in the surface treatment agent of the present invention is not particularly limited, and as an example, is 1 to 50 wt%, preferably 1 to 30 wt%, and more preferably 1 to 10 wt%, with respect to an introduced amount of the temperature-responsive polymer. In a case where the introduced amount is less than 1 wt%, the cell adhesiveness is not sufficiently exhibited, and in a case where the introduced amount is greater than 50 wt%, cell recovery properties by a cooling treatment decrease.

By applying the surface treatment agent of the present invention to the substrate and drying the surface treatment agent, it is possible to form a film containing a polymer compound of the present invention on the surface of the substrate. The type of substrate is not particularly limited, and examples thereof include polyethylene, polypropylene, polyolefin, an acrylic polymer, a methacrylic acid-based polymer, silicone rubber, polystyrene, polyethylene terephthalate, polycarbonate, a metal, ceramics, and glass. The substrate may be a mixture or a copolymer of the materials, or may contain an additive such as a plasticizer. The film may be a single layer, or may have a multi-layer structure. A substrate containing a material such as polyolefin, polystyrene, and polyethylene terephthalate is easily subjected to molding processing. In addition, the film-shaped substrate may have a thickness at which flexibility for enabling molding processing or a culture operation is obtained, and as an example, is 10 to 500 $\mu$m, and preferably 20 to 300 $\mu$m. It is preferable that the thickness of a film-shaped cell culture substrate is 10 to 500 $\mu$m.

In addition, the shape of the substrate is not particularly limited, and examples thereof include not only a plate shape, a film shape, a bead shape, and a fiber shape, but also holes, grooves, protrusions, or the like, which are provided on the plate-shaped substrate. A molding method of the film is not particularly limited, and as an example, various generally known methods such as brush coating, dip coating, spin coating, bar coating, flow coating, spray coating, roll coating, air knife coating, and blade coating can be used. The polymer solution may be applied to only one surface, or may be applied to the entire surface of the substrate. In addition, a drying method may be natural drying, or may be heating drying. The film thickness is not particularly limited, and as an example, is 1 nm to 100 $\mu$m, 10 nm to 1 $\mu$m, and the like, preferably 20 nm to 1 $\mu$m or 40 nm to 1 $\mu$m, and more preferably 20 nm to 50 nm.

The film-shaped cell culture substrate of the present invention can be used by being spread on a petri dish or a plate, or can be used in the form of a cell culture bag by welding the film.

A cell culture substrate obtained by applying the surface treatment agent of the present invention to the film-shaped substrate and drying the surface treatment agent is preferable for mass cell culture. Since a massive number of cells are required for biogenetic pharmaceuticals containing cells as a raw material, an increase in the size and a decrease in the cost are required for the temperature-responsive cell culture substrate. The general temperature-responsive cell culture substrate is in the shape of a plastic petri dish, and has a small surface area, which is not suitable for the mass cell culture. In addition, the general temperature-responsive cell culture substrate has a low production efficiency, and is not suitable for mass production. Therefore, there has been a demand for a temperature-responsive culture substrate with a large size and a high production efficiency. Since the film-shaped substrate is easily increased in the size and is also excellent in the production efficiency, the demand described above is satisfied.

A substrate having a surface covered with the film of the present invention can be used as the cell culture substrate. The cell that can be applied to the cell culture substrate of the present invention is not particularly limited, and as an example, a mesenchymal stem cell, a Chinese hamster ovary-derived CHO cell, a mouse connective tissue L929 cell, a human embryonic kidney-derived HEK293 cell, a human cervical cancer-derived HeLa cell, an epithelial cell or an endothelial cell configuring each tissue or organ in the body, a skeletal muscle cell, a plain muscle cell, and a cardiac muscle cell exhibiting contractibleness, a neuron cell, a glial cell, and a fibroblastic cell configuring a nerve system, a hepatic parenchymal cell, a hepatic nonparenchymal cell, and a fat cell involved in the metabolism in the body, a stem cell existing in various tissues, as a cell having differentiation potency, a cell induced to be differentiated therefrom, and the like can be used. In addition, a cell included in blood, lymph, cerebrospinal fluid, sputum, and urine or excrement, microorganisms, viruses, and protozoa existing in the body or in the environment, and the like can be exemplified.

In order to peel off proliferative cells from the cell culture substrate after culture, it is sufficient to change the ambient temperature to a temperature lower than LCST of the temperature-responsive polymer, preferably a temperature or lower that is 10°C lower than LCST, and culture medium replacement with a cooled culture medium or storage in a cold place can be exemplified. The culture medium replacement with the cooled culture medium is not particularly limited, and a method for taking out a warm culture medium with a pipette, and then, pouring a cooled culture medium is exemplified. The culture medium replacement can be performed in a culture liquid in which the cells have been cultured or in other culture medium solutions, which can be selected in accordance with the purpose.

A cooling time of the present invention is not particularly limited, and is preferably within 60 minutes, in order to reduce a damage to the cell by cooling.

**Examples**

Hereinafter, Examples of the present invention will be described, but the present invention is not limited to Examples described below. Note that, unless otherwise noted, a commercially available product was used as a reagent.

<Composition of Polymer>

The composition of a polymer was obtained by proton nuclear magnetic resonance ($^1$H-NMR) spectrometry using a nuclear magnetic resonance measuring device (Product Name: JNM-ECZ400S/L1, manufactured by JEOL Ltd.).

<Molecular Weight and Molecular Weight Distribution of Polymer>

A weight average molecular weight (Mw), a number average molecular weight (Mn), and a molecular weight distribution (Mw/Mn) were measured by gel permeation chromatography (GPC). As a GPC device, HLC-8320GPC, manufactured by Tosoh Corporation, was used, and as a column, two TSKgel Super AWM-H, manufactured by Tosoh Corporation, were used. The measurement was performed by setting a column temperature to 40°C, and by using N,N-dimethyl formamide containing 10 mM of phosphate and 10 mM of lithium bromide as an eluent. The measurement was performed by preparing a measurement sample at 1.0 mg/mL. As a molecular weight calibration curve, polymethyl methacrylate (manufactured by Polymer Laboratories Ltd.) with a known molecular weight was used.

<Measurement of Film Thickness>

A film thickness was measured by using a microspectroscopic film thickness gauge (Product Name: OPTM-A1, manufactured by Otsuka Electronics Co., Ltd.). In a produced culture substrate, the film thickness was measured at 51 points in a range of Φ48 mm for Examples 1 to 7 and Comparative Examples 1 to 4, the film thickness was measured at 81 points in a range of 40 mm $\times$ 40 mm for Examples 8 to 15 and Comparative Examples 5 to 7, and the average value was obtained.

<Measurement of Number of Cells>

10 $\mu$L of a cell suspension was added to a slide for measuring the number of cells (Product Name: Countess Cell Counting Chamber Slid, manufactured by Thermo Fisher Scientific Inc.), and the number of cells was measured by using an automatic cell counter (Product Name: CountessR II, manufactured by Thermo Fisher Scientific Inc.).

Example 1

A temperature-responsive polymer A, which is a triblock copolymer containing 2-methoxy ethyl acrylate (MEA), n-butyl acrylate (BA), and N-isopropyl acrylamide (IPAAm), and has a composition ratio (a molar ratio) of MEA/BA/IPAAm = 5/30/65, a number average molecular weight Mn of 128000, and a molecular weight distribution Mw/Mn of 1.5, was prepared. A lower critical solution temperature of the temperature-responsive polymer A was 32°C. In addition, a cell-adhesive polymer a, which is a random copolymer containing carboxy styrene (CSt, pKa = 4.20) as the constituent (A) and styrene (St, HLB Value = 0) as the constituent (B), and has a composition ratio (a molar ratio) of CSt/St = 33/67, a number average molecular weight Mn of 106000, and a molecular weight distribution Mw/Mn of 1.8, was prepared. 0.12 g of the temperature-responsive polymer A, 0.0012 g of the cell-adhesive polymer a, and 9.8788 g of 1-methoxy-2-propanol, as a solvent, were added to a glass vessel, were stirred, and then, were left to stand overnight to dissolve each polymer, and a surface treatment agent 1 was prepared.

100 $\mu$L of the surface treatment agent 1 was dropped onto 60 mm of a polystyrene dish for IWAKI suspension culture, and was subjected to spin coating at 3000 rpm for 60 seconds to prepare a cell culture substrate covered with the surface treatment agent 1. A film thickness was 46 nm. $1.0 \times 10^5$ cells of human bone marrow-derived mesenchymal stem cells (Product Code: PT-2501, manufactured by Lonza K.K.) were seeded on this cell culture substrate, and were cultured at 37°C and a $CO_2$ concentration of 5%. As a culture liquid, a Dulbecco modified Eagle's minimal essential medium (10 vol% FBS/DMEM) containing 10 vol% of fetal bovine serum (produced in Colombia) was used. After culture for 6 days, the culture liquid was removed, and a culture liquid cooled to 4°C was newly added, and was cooled at a room temperature for 20 minutes. After 20 minutes, the cells peeled off with a pipette were recovered, and the number of cells was measured. Further, the cells remaining on the culture substrate were recovered by using trypsin, and the total number of cells was measured. The total number of cells was $2.9 \times 10^5$ cells, and a cell recovery rate by a cooling treatment was 99%.

Example 2

0.12 g of the temperature-responsive polymer A, 0.006 g of the cell-adhesive polymer a, and 9.874 g of the 1-methoxy-2-propanol, as a solvent, were added to a glass vessel, were stirred, and then, were left to stand overnight to dissolve each polymer, and a surface treatment agent 2 was prepared.

100 $\mu$L of the surface treatment agent 2 was dropped onto 60 mm of a polystyrene dish for IWAKI suspension culture, and was subjected to spin coating at 3000 rpm for 60 seconds to prepare a cell culture substrate covered with the surface treatment agent 2. A film thickness was 48 nm. Human bone marrow-derived mesenchymal stem cells were cultured by the same method as that in Example 1, except that this cell culture substrate was used. The total number of cells was $2.8 \times 10^5$ cells, and the cell recovery rate by the cooling treatment was 95%.

Example 3

0.12 g of the temperature-responsive polymer A, 0.012 g of the cell-adhesive polymer a, and 9.868 g of the 1-methoxy-2-propanol, as a solvent, were added to a glass vessel, were stirred, and then, were left to stand overnight to dissolve each polymer, and a surface treatment agent 3 was prepared.

100 $\mu$L of the surface treatment agent 3 was dropped onto 60 mm of a polystyrene dish for IWAKI suspension culture, and was subjected to spin coating at 3000 rpm for 60 seconds to prepare a cell culture substrate covered with the surface treatment agent 3. A film thickness was 51 nm. Human bone marrow-derived mesenchymal stem cells were cultured by the same method as that in Example 1, except that this cell culture substrate was used. The total number of cells was $2.8 \times 10^5$ cells, and the cell recovery rate by the cooling treatment was 91%.

Example 4

0.3 g of the temperature-responsive polymer A, 0.03 g of the cell-adhesive polymer a, and 9.67 g of the 1-methoxy-2-propanol, as a solvent, were added to a glass vessel, were stirred, and then, were left to stand overnight to dissolve each polymer, and a surface treatment agent 4 was prepared.

100 $\mu$L of the surface treatment agent 4 was dropped onto 60 mm of a polystyrene dish for IWAKI suspension culture, and was subjected to spin coating at 3000 rpm for 60 seconds to prepare a cell culture substrate covered with the surface treatment agent 4. A film thickness was 167 nm. Human bone marrow-derived mesenchymal stem cells were cultured by the same method as that in Example 1, except that this cell culture substrate was used. The total number of cells was $2.4 \times 10^5$ cells, and the cell recovery rate by the cooling treatment was 63%.

Example 5

A temperature-responsive polymer B, which is a triblock copolymer containing n-butyl acrylate (BA) and N-isopropyl acrylamide (IPAAm), and has a composition ratio (a molar ratio) of BA/IPAAm = 35/65, a number average molecular weight Mn of 103000, and a molecular weight distribution Mw/Mn of 1.5, was prepared. A lower critical solution temperature of the temperature-responsive polymer B was 32°C. 0.12 g of the temperature-responsive polymer B, 0.0012 g of the cell-adhesive polymer a, and 9.8788 g of the 1-methoxy-2-propanol, as a solvent, were added to a glass vessel, were stirred, and then, were left to stand overnight to dissolve each polymer, and a surface treatment agent 5 was prepared.

100 $\mu$L of the surface treatment agent 5 was dropped onto 60 mm of a polystyrene dish for IWAKI suspension culture, and was subjected to spin coating at 3000 rpm for 60 seconds to prepare a cell culture substrate covered with the surface treatment agent 5. A film thickness was 48 nm. Human bone marrow-derived mesenchymal stem cells were cultured by the same method as that in Example 1, except that this cell culture substrate was used. The total number of cells was $2.6 \times 10^5$ cells, and the cell recovery rate by the cooling treatment was 50%.

Example 6

0.12 g of the temperature-responsive polymer B, 0.006 g of the cell-adhesive polymer a, and 9.874 g of the 1-methoxy-2-propanol, as a solvent, were added to a glass vessel, were stirred, and then, were left to stand overnight to dissolve each polymer, and a surface treatment agent 6 was prepared.

100 μL of the surface treatment agent 6 was dropped onto 60 mm of a polystyrene dish for IWAKI suspension culture, and was subjected to spin coating at 3000 rpm for 60 seconds to prepare a cell culture substrate covered with the surface treatment agent 6. A film thickness was 50 nm. Human bone marrow-derived mesenchymal stem cells were cultured by the same method as that in Example 1, except that this cell culture substrate was used. The total number of cells was $2.6 \times 10^5$ cells, and the cell recovery rate by the cooling treatment was 47%.

Example 7

0.3 g of the temperature-responsive polymer B, 0.03 g of the cell-adhesive polymer a, and 9.67 g of the 1-methoxy-2-propanol, as a solvent, were added to a glass vessel, were stirred, and then, were left to stand overnight to dissolve each polymer, and a surface treatment agent 7 was prepared.

100 μL of the surface treatment agent 7 was dropped onto 60 mm of a polystyrene dish for IWAKI suspension culture, and was subjected to spin coating at 3000 rpm for 60 seconds to prepare a cell culture substrate covered with the surface treatment agent 7. A film thickness was 158 nm. Human bone marrow-derived mesenchymal stem cells were cultured by the same method as that in Example 1, except that this cell culture substrate was used. The total number of cells was $2.6 \times 10^5$ cells, and the cell recovery rate by the cooling treatment was 38%.

Comparative Example 1

0.10 g of the temperature-responsive polymer A and 9.90 g of the 1-methoxy-2-propanol, as a solvent, were added to a glass vessel, were stirred, and then, were left to stand overnight to dissolve each polymer, and a surface treatment agent 8 was prepared.

100 μL of the surface treatment agent 8 was dropped onto 60 mm of a polystyrene dish for IWAKI suspension culture, and was subjected to spin coating at 3000 rpm for 60 seconds to prepare a cell culture substrate covered with the surface treatment agent 8. A film thickness was 37 nm. Human bone marrow-derived mesenchymal stem cells were cultured by the same method as that in Example 1, except that this cell culture substrate was used. The total number of cells was $0.03 \times 10^5$ cells, and the cell recovery rate was 99%.

Comparative Example 2

0.10 g of the cell-adhesive polymer a and 9.90 g of the 1-methoxy-2-propanol, as a solvent, were added to a glass vessel, were stirred, and then, were left to stand overnight to dissolve each polymer, and a surface treatment agent 9 was prepared.

100 μL of the surface treatment agent 9 was dropped onto 60 mm of a polystyrene dish for IWAKI suspension culture, and was subjected to spin coating at 3000 rpm for 60 seconds to prepare a cell culture substrate covered with the surface treatment agent 9. A film thickness was 87 nm. Human bone marrow-derived mesenchymal stem cells were cultured by the same method as that in Example 1, except that this cell culture substrate was used. The total number of cells was $2.8 \times 10^5$ cells, and the cell recovery rate was 3%.

Comparative Example 3

A cell-adhesive polymer b, which is a random copolymer containing carboxy styrene (CSt, pKa = 4.20) as the constituent (A) and n-butyl acrylate (BA, HLB Value = 6.9) as a substitute component of the constituent (B), and has a composition ratio (a molar ratio) of CSt/BA = 39/61, a number average molecular weight Mn of 70000, and a molecular weight distribution Mw/Mn of 1.6, was prepared. 0.10 g of the temperature-responsive polymer A, 0.010 g of the cell-adhesive polymer b, and 9.890 g of the 1-methoxy-2-propanol, as a solvent, were added to a glass vessel, were stirred, and then, were left to stand overnight to dissolve each polymer, and a surface treatment agent 10 was prepared.

100 μL of the surface treatment agent 10 was dropped onto 60 mm of a polystyrene dish for IWAKI suspension culture, and was subjected to spin coating at 3000 rpm for 60 seconds to prepare a cell culture substrate covered with the surface treatment agent 10. A film thickness was 46 nm. Human bone marrow-derived mesenchymal stem cells were cultured by the same method as that in Example 1, except that this cell culture substrate was used. The total number of cells was $1.3 \times 10^5$ cells, and the cell recovery rate by the cooling treatment was 98%.

Comparative Example 4

0.10 g of the temperature-responsive polymer B and 9.90 g of the 1-methoxy-2-propanol, as a solvent, were added to a glass vessel, were stirred, and then, were left to stand overnight to dissolve each polymer, and a surface treatment agent 11 was prepared.

100 $\mu$L of the surface treatment agent 11 was dropped onto 60 mm of a polystyrene dish for IWAKI suspension culture, and was subjected to spin coating at 3000 rpm for 60 seconds to prepare a cell culture substrate covered with the surface treatment agent 11. A film thickness was 38 nm. Human bone marrow-derived mesenchymal stem cells were cultured by the same method as that in Example 1, except that this cell culture substrate was used. The total number of cells was $0.05 \times 10^5$ cells, and the cell recovery rate was 99%.

[Table 1]

|  | Temperature-responsive polymer | | Cell-adhesive polymer | | | | Concentration of temperature-responsive polymer [wt%] | Concentration of cell-adhesive polymer [wt%] | Film thickness [nm] | Substrate | Total number of cells [$10^5$ cells] | Cell recovery rate [%] |
|---|---|---|---|---|---|---|---|---|---|---|---|---|
|  |  | LCST[°C] | (A) | pKa | (B) | HLB |  |  |  |  |  |  |
| Example 1 | MEA-BA-IPAAm | 32 | CSt | 4.20 | St | 0.0 | 1.20 | 0.012 | 46 | Polystyrene dish | 2.9 | 99 |
| Example 2 | MEA-BA-IPAAm | 32 | CSt | 4.20 | St | 0.0 | 1.20 | 0.06 | 48 | Polystyrene dish | 2.8 | 95 |
| Example 3 | MEA-BA-IPAAm | 32 | CSt | 4.20 | St | 0.0 | 1.20 | 0.12 | 51 | Polystyrene dish | 2.8 | 91 |
| Example 4 | MEA-BA-IPAAm | 32 | CSt | 4.20 | St | 0.0 | 3.00 | 0.30 | 167 | Polystyrene dish | 2.4 | 63 |
| Example 5 | BA-IPAAm | 32 | CSt | 4.20 | St | 0.0 | 1.20 | 0.012 | 48 | Polystyrene dish | 2.6 | 50 |
| Example 6 | BA-IPAAm | 32 | CSt | 4.20 | St | 0.0 | 1.20 | 0.06 | 50 | Polystyrene dish | 2.6 | 47 |
| Example 7 | BA-IPAAm | 32 | CSt | 4.20 | St | 0.0 | 3.00 | 0.30 | 158 | Polystyrene dish | 2.6 | 38 |
| Comparative Example 1 | MEA-BA-IPAAm | 32 | -- | -- | -- | -- | 1.00 | 0.00 | 37 | Polystyrene dish | 0.03 | 99 |
| Comparative Example 2 | -- | -- | CSt | 4.20 | St | 0.0 | 0.00 | 1.00 | 87 | Polystyrene dish | 2.8 | 3 |
| Comparative Example 3 | MEA-BA-IPAAm | 32 | CSt | 4.20 | BA | 6.9 | 1.00 | 0.10 | 46 | Polystyrene dish | 1.3 | 98 |
| Comparative Example 4 | BA-IPAAm | 32 | -- | -- | -- | -- | 1.00 | 0.00 | 38 | Polystyrene dish | 0.05 | 99 |

EP 4 299 716 B1

Example 8

A polymer solution 1 (the surface treatment agent 1) was applied to a linear low-density polyethylene film (LLDPE, manufactured by TOSHIN CHEMICAL INDUSTRY CO., LTD.) with a thickness of 100 $\mu$m by using a bar coater (OSP-05, manufactured by OSG SYSTEM PRODUCTS Co., LTD.), and was air-dried to prepare a cell culture substrate 12. A film thickness was 45 nm. The cell culture substrate 12 cut into the shape of a circle was provided on an untreated dish for suspended cells (1010-060, manufactured by AGC TECHNO GLASS CO., LTD.), and $1.0 \times 10^5$ cells of human bone marrow-derived mesenchymal stem cells (Product Code: PT-2501, manufactured by Lonza K.K.) were seeded, and were cultured at 37°C and a $CO_2$ concentration of 5%. As a culture liquid, a Dulbecco modified Eagle's minimal essential medium (10 vol% FBS/DMEM) containing 10 vol% of a fetal bovine serum (produced in Colombia) was used. After culture for 7 days, the culture liquid was removed, and a culture liquid cooled to 4°C was newly added, and was cooled at a room temperature for 10 minutes. After 10 minutes, the cells peeled off with a pipette were recovered, and the number of cells was measured. Further, the cells remaining on the culture substrate were recovered by using trypsin, and the total number of cells was measured. The total number of cells was $3.7 \times 10^5$ cells, and the cell recovery rate by the cooling treatment for 10 minutes was 100%.

Example 9

A polymer solution 2 (the surface treatment agent 2) was applied to a linear low-density polyethylene film (LLDPE, manufactured by TOSHIN CHEMICAL INDUSTRY CO., LTD.) with a thickness of 100 $\mu$m by using a bar coater (OSP-05, manufactured by OSG SYSTEM PRODUCTS Co., LTD.), and was air-dried to prepare a cell culture substrate 13. A film thickness was 48 nm. The cell culture substrate 13 cut into the shape of a circle was provided on an untreated dish for suspended cells (1010-060, manufactured by AGC TECHNO GLASS CO., LTD.), and human bone marrow-derived mesenchymal stem cells were cultured by the same method as that in Example 8. The total number of cells was $4.2 \times 10^5$ cells, and the cell recovery rate by the cooling treatment for 30 minutes was 100%.

Example 10

A polymer solution 3 (the surface treatment agent 3) was applied to a linear low-density polyethylene film (LLDPE, manufactured by TOSHIN CHEMICAL INDUSTRY CO., LTD.) with a thickness of 100 $\mu$m by using a bar coater (OSP-05, manufactured by OSG SYSTEM PRODUCTS Co., LTD.), and was air-dried to prepare a cell culture substrate 14. A film thickness was 47 nm. The cell culture substrate 14 cut into the shape of a circle was provided on an untreated dish for suspended cells (1010-060, manufactured by AGC TECHNO GLASS CO., LTD.), and human bone marrow-derived mesenchymal stem cells were cultured by the same method as that in Example 8. The total number of cells was $4.3 \times 10^5$ cells, and the cell recovery rate by the cooling treatment for 60 minutes was 94%.

Example 11

0.12 g of the temperature-responsive polymer A, 0.036 g of the cell-adhesive polymer a, and 9.844 g of the 1-methoxy-2-propanol, as a solvent, were added to a glass vessel, were stirred, and then, were left to stand overnight to dissolve each polymer, and a surface treatment agent 12 was prepared. A polymer solution 12 was applied to a linear low-density polyethylene film (LLDPE, manufactured by TOSHIN CHEMICAL INDUSTRY CO., LTD.) with a thickness of 100 $\mu$m by using a bar coater (OSP-05, manufactured by OSG SYSTEM PRODUCTS Co., LTD.), and was air-dried to prepare a cell culture substrate 15. A film thickness was 58 nm. The cell culture substrate 15 cut into the shape of a circle was provided on an untreated dish for suspended cells (1010-060, manufactured by AGC TECHNO GLASS CO., LTD.), and human bone marrow-derived mesenchymal stem cells were cultured by the same method as that in Example 8. The total number of cells was $3.8 \times 10^5$ cells, and the cell recovery rate by the cooling treatment for 60 minutes was 71%.

Example 12

0.12 g of the temperature-responsive polymer A, 0.060 g of the cell-adhesive polymer a, and 9.820 g of the 1-methoxy-2-propanol, as a solvent, were added to a glass vessel, were stirred, and then, were left to stand overnight to dissolve each polymer, and a surface treatment agent 13 was prepared. A polymer solution 13 was applied to a linear low-density polyethylene film (LLDPE, manufactured by TOSHIN CHEMICAL INDUSTRY CO., LTD.) with a thickness of 100 $\mu$m by using a bar coater (OSP-05, manufactured by OSG SYSTEM PRODUCTS Co., LTD.), and was air-dried to prepare a cell culture substrate 16. A film thickness was 86 nm. The cell culture substrate 16 cut into the shape of a circle was provided on an untreated dish for suspended cells (1010-060, manufactured by AGC TECHNO GLASS CO., LTD.), and human bone marrow-derived mesenchymal stem cells were cultured by the same method as that in Example 8. The total number of cells was $3.6 \times 10^5$ cells, and the cell recovery rate by the cooling treatment for 60 minutes was 69%.

Example 13

0.30 g of the temperature-responsive polymer A, 0.030 g of the cell-adhesive polymer a, and 9.670 g of the 1-methoxy-2-propanol, as a solvent, were added to a glass vessel, were stirred, and were left to stand overnight to dissolve each polymer, and a surface treatment agent 14 was prepared. A polymer solution 14 was applied to a linear low-density polyethylene film (LLDPE, manufactured by TOSHIN CHEMICAL INDUSTRY CO., LTD.) with a thickness of 100 $\mu$m by using a bar coater (OSP-05, manufactured by OSG SYSTEM PRODUCTS Co., LTD.), and was air-dried to prepare a cell culture substrate 17. A film thickness was 145 nm. The cell culture substrate 17 cut into the shape of a circle was provided on an untreated dish for suspended cells (1010-060, manufactured by AGC TECHNO GLASS CO., LTD.), and human bone marrow-derived mesenchymal stem cells were cultured by the same method as that in Example 8. The total number of cells was $2.7 \times 10^5$ cells, and the cell recovery rate by the cooling treatment for 30 minutes was 94%.

Example 14

The polymer solution 2 (the surface treatment agent 2) was applied to a polyethylene terephthalate film (Lumirror T60, manufactured by Toray Industries, Inc.) with a thickness of 25 $\mu$m by using a bar coater (OSP-05, manufactured by OSG SYSTEM PRODUCTS Co., LTD.), and was air-dried to prepare a cell culture substrate 18. A film thickness was 57 nm. The cell culture substrate 18 cut into the shape of a circle was provided on an untreated dish for suspended cells (1010-060, manufactured by AGC TECHNO GLASS CO., LTD.), and human bone marrow-derived mesenchymal stem cells were cultured by the same method as that in Example 8. The total number of cells was $4.0 \times 10^5$ cells, and the cell recovery rate by the cooling treatment for 20 minutes was 97%.

Example 15

A polymer solution 5 (the surface treatment agent 5) was applied to a linear low-density polyethylene film (LLDPE, manufactured by TOSHIN CHEMICAL INDUSTRY CO., LTD.) with a thickness of 100 $\mu$m by using a bar coater (OSP-05, manufactured by OSG SYSTEM PRODUCTS Co., LTD.), and was air-dried to prepare a cell culture substrate 19. A film thickness was 45 nm. The cell culture substrate 19 cut into the shape of a circle was provided on an untreated dish for suspended cells (1010-060, manufactured by AGC TECHNO GLASS CO., LTD.), and human bone marrow-derived mesenchymal stem cells were cultured by the same method as that in Example 8. The total number of cells was $3.7 \times 10^5$ cells, and the cell recovery rate by the cooling treatment for 20 minutes was 55%.

Comparative Example 5

A polymer solution 8 (the surface treatment agent 8) was applied to a linear low-density polyethylene film (LLDPE, manufactured by TOSHIN CHEMICAL INDUSTRY CO., LTD.) with a thickness of 100 $\mu$m by using a bar coater (OSP-05, manufactured by OSG SYSTEM PRODUCTS Co., LTD.), and was air-dried to prepare a cell culture substrate 20. A film thickness was 46 nm. The cell culture substrate 20 cut into the shape of a circle was provided on an untreated dish for suspended cells (1010-060, manufactured by AGC TECHNO GLASS CO., LTD.), and human bone marrow-derived mesenchymal stem cells were cultured by the same method as that in Example 8. The total number of cells was $0.13 \times 10^5$ cells, and the cell recovery rate by the cooling treatment for 10 minutes was 100%.

Comparative Example 6

A polymer solution 9 (the surface treatment agent 9) was applied to a linear low-density polyethylene film (LLDPE, manufactured by TOSHIN CHEMICAL INDUSTRY CO., LTD.) with a thickness of 100 $\mu$m by using a bar coater (OSP-05, manufactured by OSG SYSTEM PRODUCTS Co., LTD.), and was air-dried to prepare a cell culture substrate 21. A film thickness was 49 nm. The cell culture substrate 21 cut into the shape of a circle was provided on an untreated dish for suspended cells (1010-060, manufactured by AGC TECHNO GLASS CO., LTD.), and human bone marrow-derived mesenchymal stem cells were cultured by the same method as that in Example 8. The total number of cells was $3.4 \times 10^5$ cells, and the cell recovery rate by the cooling treatment for 60 minutes was 18%.

Comparative Example 7

A polymer solution 11 (the surface treatment agent 11) was applied to a linear low-density polyethylene film (LLDPE, manufactured by TOSHIN CHEMICAL INDUSTRY CO., LTD.) with a thickness of 100 $\mu$m by using a bar coater (OSP-05, manufactured by OSG SYSTEM PRODUCTS Co., LTD.), and was air-dried to prepare a cell culture substrate 22. A film thickness was 47 nm. The cell culture substrate 22 cut into the shape of a circle was provided on an untreated dish for suspended cells (1010-060, manufactured by AGC TECHNO GLASS CO., LTD.), and human bone marrow-derived mesenchymal stem cells were cultured by the same method as that in Example 8. The total number of cells was $0.05 \times 10^5$ cells, and the cell recovery rate by the cooling treatment for 10 minutes was 100%.

[Table 2]

| | Temperature-responsive polymer | | Cell-adhesive polymer | | | | Concentration of temperature-responsive polymer [wt%] | Concentration of cell-adhesive polymer [wt%] | Film thickness [nm] | Substrate | Total number of cells [$10^5$ cells] | Cell recovery rate [%] | Cooling time [min] |
|---|---|---|---|---|---|---|---|---|---|---|---|---|---|
| | | LCST[°C] | (A) | pKa | (B) | HLB | | | | | | | |
| Example 8 | MEA-BA-IPAAm | 32 | CSt | 4.20 | St | 0.0 | 1.20 | 0.012 | 45 | LLDPE film | 3.7 | 100 | 10 |
| Example 9 | MEA-BA-IPAAm | 32 | CSt | 4.20 | St | 0.0 | 1.20 | 0.06 | 48 | LLDPE film | 4.2 | 100 | 30 |
| Example 10 | MEA-BA-IPAAm | 32 | CSt | 4.20 | St | 0.0 | 1.20 | 0.12 | 47 | LLDPE film | 4.3 | 94 | 60 |
| Example 11 | MEA-BA-IPAAm | 32 | CSt | 4.20 | St | 0.0 | 1.20 | 0.36 | 58 | LLDPE film | 3.8 | 71 | 60 |
| Example 12 | MEA-BA-IPAAm | 32 | CSt | 4.20 | St | 0.0 | 1.20 | 0.60 | 86 | LLDPE film | 3.6 | 69 | 60 |
| Example 13 | MEA-BA-IPAAm | 32 | CSt | 4.20 | St | 0.0 | 3.00 | 0.30 | 145 | LLDPE film | 2.7 | 94 | 30 |
| Example 14 | MEA-BA-IPAAm | 32 | CSt | 4.20 | St | 0.0 | 1.20 | 0.06 | 57 | PET film | 4.0 | 97 | 20 |
| Example 15 | BA-IPAAm | 32 | CSt | 4.20 | St | 0.0 | 1.20 | 0.012 | 45 | LLDPE film | 3.7 | 55 | 20 |
| Comparative Example 5 | MEA-BA-IPAAm | 32 | -- | -- | -- | -- | 1.00 | 0.00 | 46 | LLDPE film | 0.13 | 100 | 10 |
| Comparative Example 6 | -- | -- | CSt | 4.20 | St | 0.0 | 0.00 | 1.00 | 49 | LLDPE film | 3.4 | 18 | 60 |
| Comparative Example 7 | BA-IPAAm | 32 | -- | -- | -- | -- | 1.00 | 0.00 | 47 | LLDPE film | 0.05 | 100 | 10 |

**Claims**

1.  A surface treatment agent, comprising:

    a temperature-responsive polymer; and
    a cell-adhesive polymer including (A) and (B) described below;

    (A) a monomer having a functional group exhibiting acidity, and
    (B) a monomer having a HLB value (a Griffin's method) in a range of 0 to 5.0.

2.  The surface treatment agent according to claim 1,
    wherein an introduced amount of the cell-adhesive polymer is 1 to 50 wt% with respect to an introduced amount of the temperature-responsive polymer.

3.  The surface treatment agent according to claim 1 or 2,
    wherein the temperature-responsive polymer exhibits a lower critical solution temperature with respect to water in a range of 0°C to 50°C.

4.  The surface treatment agent according to any one of claims 1 to 3,
    wherein the temperature-responsive polymer is a block copolymer including repeating units of three or more components.

5.  The surface treatment agent according to any one of claims 1 to 4,
    wherein the functional group exhibiting acidity of the constituent (A) is selected from a hydroxy group, a carboxy group, a sulfo group, and a phosphate group.

6.  The surface treatment agent according to any one of claims 1 to 5,
    wherein an acidity dissociation constant pKa of the functional group exhibiting acidity of the constituent (A) is -5.0 to 6.0.

7.  The surface treatment agent according to any one of claims 1 to 6,
    wherein a concentration of the temperature-responsive polymer in the surface treatment agent is 0.1 to 5.0 wt%.

8.  A film obtained by applying the surface treatment agent according to any one of claims 1 to 7 to a substrate.

9.  The film according to claim 8,
    wherein a film thickness is 1 to 1000 nm.

10. A cell culture substrate having a surface covered with the film according to claim 8 or 9.

11. The cell culture substrate according to claim 10,
    wherein the cell culture substrate is in the shape of a film.

12. A method for manufacturing the cell culture substrate according to claim 10 or 11, the method comprising
    a step of applying the surface treatment agent to the substrate.


**Patentansprüche**

1.  Oberflächenbehandlungsmittel, umfassend:

    ein temperaturreaktives Polymer; und
    ein zelladhäsives Polymer, das die nachfolgend beschriebenen (A) und (B) umfasst;

    (A) ein Monomer, das eine funktionelle Gruppe aufweist, die Säure aufweist, und
    (B) ein Monomer, das einen HLB-Wert (Griffin-Methode) in einem Bereich von 0 bis 5,0 aufweist.

2.  Oberflächenbehandlungsmittel nach Anspruch 1,

wobei eine eingeführte Menge des zelladhäsiven Polymers, bezogen auf eine eingeführte Menge des temperaturreaktiven Polymers, 1 bis 50 Gew.-% beträgt.

3.  Oberflächenbehandlungsmittel nach Anspruch 1 oder 2, wobei das temperaturreaktive Polymer, bezogen auf Wasser, eine untere kritische Lösungstemperatur in einem Bereich von 0°C bis 50°C aufweist.

4.  Oberflächenbehandlungsmittel nach einem der Ansprüche 1 bis 3, wobei das temperaturreaktive Polymer ein Block-Copolymer ist, das sich wiederholende Einheiten aus drei oder mehr Komponenten umfasst.

5.  Oberflächenbehandlungsmittel nach einem der Ansprüche 1 bis 4, wobei die Säure aufweisende funktionelle Gruppe des Bestandteils (A) ausgewählt ist aus einer Hydroxy-Gruppe, einer Carboxy-Gruppe, einer Sulfo-Gruppe und einer Phosphat-Gruppe.

6.  Oberflächenbehandlungsmittel nach einem der Ansprüche 1 bis 5, wobei eine Säuredissoziationskonstante pKa der Säure aufweisenden funktionellen Gruppe des Bestandteils (A) -5,0 bis 6,0 beträgt.

7.  Oberflächenbehandlungsmittel nach einem der Ansprüche 1 bis 6, wobei eine Konzentration des temperaturreaktiven Polymers in dem Oberflächenbehandlungsmittel 0,1 bis 5,0 Gew.-% beträgt.

8.  Film, der durch Aufbringen des Oberflächenbehandlungsmittels nach einem der Ansprüche 1 bis 7 auf ein Substrat erhalten wird.

9.  Film nach Anspruch 8, wobei eine Filmdicke 1 bis 1000 nm beträgt.

10. Zellkultursubstrat, das eine mit dem Film nach Anspruch 8 oder 9 bedeckte Oberfläche aufweist.

11. Zellkultursubstrat nach Anspruch 10, wobei das Zellkultursubstrat in der Form eines Films vorliegt.

12. Verfahren zur Herstellung des Zellkultursubstrats nach Anspruch 10 oder 11, wobei das Verfahren umfasst: einen Schritt des Aufbringens des Oberflächenbehandlungsmittels auf das Substrat.


**Revendications**

1.  Agent de traitement de surface, comprenant :

    un polymère sensible à la température ; et
    un polymère présentant une adhérence cellulaire contenant (A) et (B) décrits ci-dessous ;

    (A) un monomère ayant un groupe fonctionnel présentant une acidité, et
    (B) un monomère ayant un indice HLB (méthode de Griffin) situé dans la plage allant de 0 à 5,0.

2.  Agent de traitement de surface selon la revendication 1, dans lequel la quantité introduite du polymère présentant une adhérence cellulaire est de 1 à 50 % en poids par rapport à la quantité introduite du polymère sensible à la température.

3.  Agent de traitement de surface selon la revendication 1 ou 2, dans lequel le polymère sensible à la température présente une température de solution critique inférieure par rapport à l'eau située dans la plage allant de 0°C à 50°C.

4.  Agent de traitement de surface selon l'une quelconque des revendications 1 à 3, dans lequel le polymère sensible à la température est un copolymère séquencé contenant des motifs répétitifs de trois composants ou plus.

**5.** Agent de traitement de surface selon l'une quelconque des revendications 1 à 4, dans lequel le groupe fonctionnel présentant une acidité du constituant (A) est choisi parmi un groupe hydroxy, un groupe carboxy, un groupe sulfo, et un groupe phosphate.

**6.** Agent de traitement de surface selon l'une quelconque des revendications 1 à 5, dans lequel la constante de dissociation d'acidité pKa du groupe fonctionnel présentant une acidité du constituant (A) est de -5,0 à 6,0.

**7.** Agent de traitement de surface selon l'une quelconque des revendications 1 à 6, dans lequel la concentration du polymère sensible à la température dans l'agent de traitement de surface est de 0,1 à 5,0 % en poids.

**8.** Film obtenu par application de l'agent de traitement de surface selon l'une quelconque des revendications 1 à 7 à un substrat.

**9.** Film selon la revendication 8, dans lequel l'épaisseur de film est de 1 à 1 000 nm.

**10.** Substrat de culture cellulaire ayant une surface recouverte du film selon la revendication 8 ou 9.

**11.** Substrat de culture cellulaire selon la revendication 10, lequel substrat de culture cellulaire est sous la forme d'un film.

**12.** Procédé de fabrication du substrat de culture cellulaire selon la revendication 10 ou 11, le procédé comprenant une étape d'application de l'agent de traitement de surface au substrat.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020110140 A
- JP 5846584 B
- JP 6447787 B
- JP 201355907 B

**Non-patent literature cited in the description**

- **W. C. GRIFFIN**. *Journal of the Society of Cosmetic Chemists*, 1949, vol. 1, 311